(19) **Europäisches Patentamt**
**European Patent Office**
**Office européen des brevets**

(11) **EP 2 561 457 B1**

(12) **EUROPEAN PATENT SPECIFICATION**

(45) Date of publication and mention
of the grant of the patent:
**14.11.2018 Bulletin 2018/46**

(51) Int Cl.:
***G16H 50/70*** *(2018.01)*

(21) Application number: **11718202.2**

(86) International application number:
**PCT/US2011/033590**

(22) Date of filing: **22.04.2011**

(87) International publication number:
**WO 2011/133869 (27.10.2011 Gazette 2011/43)**

(54) **SYSTEM AND METHOD OF IDENTIFYING WHEN A PATIENT UNDERGOING HEMODIALYSIS IS AT INCREASED RISK OF DEATH BY A LOGISTIC REGRESSION MODEL**

SYSTEM UND VERFAHREN ZUR IDENTIFIZIERUNG EINES ERHÖHTEN RISIKOS DES TODES EINES HÄMODIALYSEPATIENTEN DURCH LOGISTISCHES REGRESSIONSMODELL

SYSTÈME ET PROCÉDÉ D'IDENTIFICATION DU FAIT QU'UN PATIENT SOUMIS À L'HÉMODIALYSE PRÉSENTE UN RISQUE ACCRU DE MORT SELON UN MODÈLE DE RÉGRESSION LOGISTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.04.2010 US 327439 P**

(43) Date of publication of application:
**27.02.2013 Bulletin 2013/09**

(73) Proprietor: **Fresenius Medical Care Holdings, Inc.
Waltham, MA 02451 (US)**

(72) Inventors:
• **KOTANKO, Peter
New York, NY 10128 (US)**
• **THIJSSEN, Stephan
New York, NY 10044 (US)**
• **USVYAT, Len
Philadelphia, PA 19123 (US)**
• **LEVIN, Nathan, W.
New York, NY 10128 (US)**

(74) Representative: **Kirkham, Nicholas Andrew et al
Graham Watt & Co. LLP
St. Botolph's House
7-9 St. Botolph's Road
Sevenoaks, Kent TN13 3AJ (GB)**

(56) References cited:
**US-A1- 2010 099 958**

• **I ANNESI ET AL: "Efficiency of the logistic regression and cox proportional hazards models in longitudinal studies", STATISTICS IN MEDICINE, vol. 8, no. 12, 1 December 1989 (1989-12-01), pages 1515-1521, XP55002154,**
• **G M CHERTOW ET AL: "Mortality after acute renal failure: Models for prognostic stratification and risk adjustment", KIDNEY INTERNATIONAL, vol. 70, 19 July 2006 (2006-07-19), pages 1120-1126, XP55002161,**
• **ABOTT ET AL: "Logistic Regression in Survival Analysis", AMERICAN JOURNAL OF EPIDEMIOLOGY, HOPKINS UNIVERSITY, BALTIMORE, vol. 121, no. 3, 1 January 1985 (1985-01-01), pages 465-471, XP009150049, ISSN: 0002-9262**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION

Despite significant advances in hemodialysis (HD) technology, the mortality risk of chronic HD patients remains well above that seen in the general population. The average life expectancy in the general population is several times higher than in dialysis patients, and the adjusted rates of all-cause mortality are substantially higher for dialysis patients than in the general population. Cardiovascular disease and infectious disease are among the leading causes of mortality, and the overall annual mortality rate in dialysis patients is about 20% in the United States. *See* United States Renal Data System, USRDS 2009 Annual Data Report, National Institutes of Health.

Current epidemiologic studies seeking to investigate the determinants of mortality risk in dialysis patients usually consider either cross-sectional baseline characteristics (*e.g.*, mean systolic blood pressure in the first 3 months after start of dialysis; serum albumin levels after 6 months) or time-dependent analyses, most commonly time-dependent Cox regression models. Patients are frequently stratified into groups based on descriptive characteristics such as tertiles. Of note, in many of these studies, the first date of dialysis is taken as the reference point.

A recent improved approach, described in U.S. Application No. 12/587,941, filed October 15, 2009, entitled "Method of Identifying When A Patient Undergoing Hemodialysis Is At Increased Risk Of Death," includes determining at least one of the patient's clinical or biochemical parameters periodically while the patient is undergoing hemodialysis treatments, and identifying a patient as having an increased risk of death if the patient has a substantial change in the rate of decline or the rate of increase of the clinical or biochemical parameter.

SUMMARY OF THE INVENTION

The present invention generally is directed to identifying a patient undergoing periodic hemodialysis treatments at increased risk of death by use of a logistic regression model.

In one embodiment, a method includes selecting one or more clinical or biochemical parameters associated with a probability of death of the patient while the patient is undergoing periodic hemodialysis treatments, and estimating the probability of death of the patient over a future time interval by a logistic regression model including model coefficients. The model coefficients are determined by analyzing data from deceased patients that were previously undergoing periodic hemodialysis treatments, the analysis including a longitudinal analysis backwards in time on the one or more clinical or biochemical parameter of the deceased patients. In certain embodiments, the future time interval can be in a range of between about one month and about six months. The patient is identified as having an increased risk of death if the probability of death of the patient is greater than a predetermined threshold probability (e.g., about 2.5% in the future time interval).

In some embodiments, the one or more clinical or biochemical parameters can include the patient's age, race, gender, diabetic status, pre- and post-dialysis systolic blood pressure (SBP), pre- and post-dialysis diastolic blood pressure (DBP), pre- and post-dialysis weight, inter-dialytic weight change, intra-dialytic change in SBP, pre-dialysis pulse pressure, serum albumin level, serum sodium level, equilibrated normalized protein catabolic rate (enPCR), eKdrt/V, transferrin saturation index (TSAT), serum creatinine level, serum bicarbonate level, erythropoietin (EPO) resistance index (ERI), neutrophil to lymphocyte ratio, the sodium gradient during dialysis, the percent change in serum albumin level in a previous time interval (*e.g.*, two months), the percent change in pre-dialysis weight in a previous time interval (*e.g.*, two months or three months), the percent change in ferritin level in a previous time interval (*e.g.*, six months), or any combinations thereof. Other parameters can be included, provided that they are associated with a risk of death of a patient undergoing periodic hemodialysis treatments. Identifying the patient as having an increased risk of death is preferably accomplished within a sufficient lead time to allow for therapeutic intervention to decrease the patient's risk of death.

In another embodiment, a computer system for identifying a patient undergoing periodic hemodialysis treatments at increased risk of death includes a user input means for determining patient data from a user, and a digital processor coupled to receive determined patient data from the input means. The digital processor executes a modeling system in working memory, wherein the modeling system selects one or more clinical or biochemical parameters associated with a probability of death of the patient while the patient is undergoing periodic hemodialysis treatments, estimates the probability of death of the patient over a future time interval by a logistic regression model including model coefficients, the model coefficients determined by analyzing data from deceased patients that were previously undergoing periodic hemodialysis treatments, the analysis including a longitudinal analysis backwards in time on the one or more clinical or biochemical parameters of the deceased patients, and identifies the patient as having an increased risk of death if the probability of death of the patient is greater than a predetermined threshold probability. The computer system also includes an output means coupled to the digital processor, the output means provides to the user the probability of death of the patient while the patient is undergoing periodic hemodialysis treatments.

This invention has many advantages, including the ability to provide a more accurate estimate of the probability of death over a future time interval for a patient undergoing periodic hemodialysis treatments.

BRIEF DESCRIPTION OF THE DRAWINGS

The foregoing will be apparent from the following more particular description of example embodiments of the invention, as illustrated in the accompanying drawings. The drawings are not necessarily to scale, emphasis instead being placed upon illustrating embodiments of the present invention.

FIG. 1 is a schematic view of a computer network in which the present invention can be implemented.
FIG. 2 is a block diagram of a computer of the network of FIG. 1.

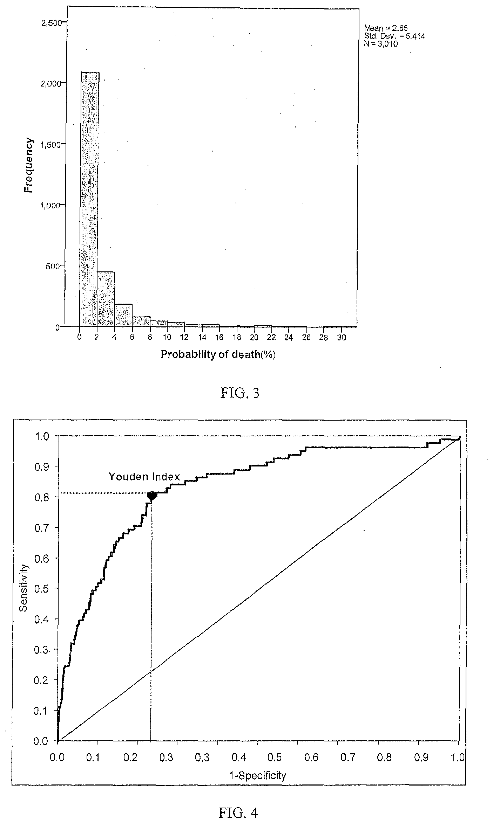

FIG. 3 is a graph of the percent frequency distribution probability of death as a function of frequency of probability estimates as obtained by a logistic regression model constructed according to this invention.
FIG. 4 is a receiver-operator characteristic curve for the logistic regression model employed to obtain the data shown in FIG. 3.

DETAILED DESCRIPTION OF THE INVENTION

The present invention discloses computer systems and methods for identifying a patient undergoing periodic hemodialysis treatments at increased risk of death according to the claims. The present method of identifying a patient undergoing periodic hemodialysis treatments at increased risk of death employs a logistic regression model. *See* D. G. Kleinbaum and M. Klein, Survival Analysis, 2nd Ed. Springer (2005). As applied herein, a logistic regression model considers the following general epidemiologic study framework: independent variables $X_1$, $X_2$, and so on up to $X_k$ are observed on a group of hemodialysis patients for whom the outcome (alive/dead) over a retrospective time period is also known. In a logistic model, the probability that a live hemodialysis patient with independent variable values of $X_1$, $X_2$ up to $X_k$ will die during a defined study period (time interval) is equal to

$$P\left(D=1\,|\,X_1,X_2,...X_k\right)=\frac{1}{1+e^{-(\alpha+\sum_{i=1}^{k}\beta_i X_i)}} \qquad (1)$$

where $\alpha$, the intercept, and $\beta_i$, the coefficients for the independent variables $X_i$, are obtained from regression of data on the group of hemodialysis patients for whom the outcome (alive/dead) over a retrospective time period is known. The retrospective time period can be in a range of about one month to about 24 months. In contrast to simple or multiple regression models, a logistic regression model yields an estimate of the probability of a binary outcome (alive/dead).
In one embodiment, the method includes selecting one or more clinical or biochemical parameters associated with a probability of death of the patient (the independent variables $X_i$) while the patient is undergoing periodic hemodialysis treatments, and estimating the probability of death of the patient over a future time interval by the logistic regression model described by Eq. 1, including model coefficients $\alpha$ and $\beta_i$. The model coefficients are determined by analyzing data from deceased patients that were previously undergoing periodic hemodialysis treatments, the analysis including a longitudinal analysis backwards in time on the one or more clinical or biochemical parameters of the deceased patients. In certain embodiments, the future time interval can be in a range of between about one month and about six months. The patient is identified as having an increased risk of death if the probability of death of the patient is greater than a predetermined threshold probability (*e.g.*, about 2.5% in the future time interval).
In some embodiments, the one or more clinical or biochemical parameters can include the patient's age, race, gender, diabetic status, pre- and post-dialysis systolic blood pressure (SBP), pre- and post-dialysis diastolic blood pressure (DBP), pre- and post-dialysis weight, inter-dialytic weight change, intra-dialytic change in SBP, pre-dialysis pulse pressure, serum albumin level, enPCR level, eKdrt/V, transferrin saturation index (TSAT), serum creatinine level, serum bicarbonate level, serum sodium level, erythropoietin (EPO) resistance index (ERI), neutrophil to lymphocyte ratio, the sodium gradient during dialysis, the percent change in serum albumin level in the previous two months, the percent change in pre-dialysis weight in the previous two months, the percent change in pre-dialysis weight in the previous three months, the percent change in ferritin level in the previous six months, or any combinations thereof. Other parameters can be included, provided that they are associated with a risk of death of a patient undergoing periodic hemodialysis treatments. For a comprehensive description of clinical and biochemical parameters for a patient undergoing periodic hemodialysis treatments, *see* J. T. Daugirdas, P. G. Blake, and T. S. Ing, Handbook of Dialysis, (2007). The eKdrt/V is the equilibrated measure of the dialysis dose, taking into account the dialysis treatment and the patient's residual kidney function. The erythropoietin (EPO) resistance index ERI = EPO dose per treatment/(post-dialysis weight*hemoglobin). Identifying the patient as having an increased risk of death is preferably accomplished within a sufficient lead time to allow for a therapeutic intervention to decrease the patient's risk of death.

In another embodiment, a computer system for identifying a patient undergoing periodic hemodialysis treatments at increased risk of death includes a user input means for determining patient data from a user, and a digital processor coupled to receive determined patient data from the input means. The digital processor executes a modeling system in working memory, wherein the modeling system selects one or more clinical or biochemical parameters associated with a probability of death of the patient while the patient is undergoing periodic hemodialysis treatments, estimates the probability of death of the patient over a future time interval by a logistic regression model including model coefficients, the model coefficients determined by analyzing data from deceased patients that were previously undergoing periodic hemodialysis treatments, the analysis including a longitudinal analysis backwards in time on the one or more clinical or biochemical parameters of the deceased patients, and identifies the patient as having an increased risk of death if the probability of death of the patient is greater than a predetermined threshold probability. The computer system also includes an output means coupled to the digital processor, the output means provides to the user the probability of death of the patient while the patient is undergoing periodic hemodialysis treatments.

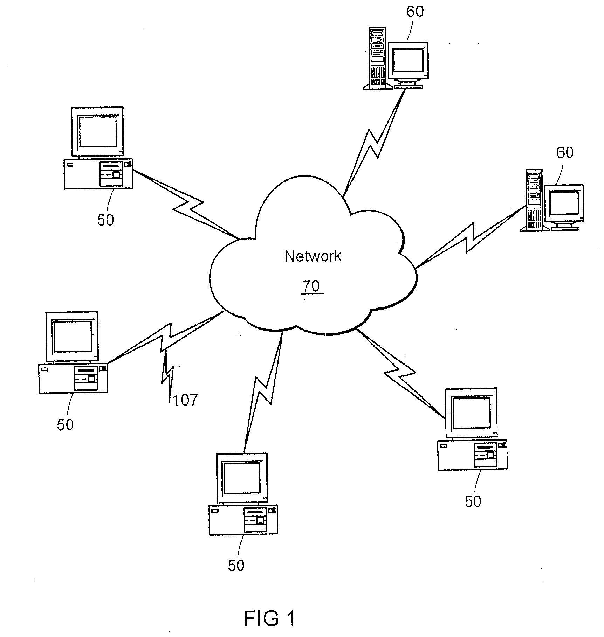

FIG. 1 illustrates a computer network or similar digital processing environment in which the present invention can be implemented.

Client computer(s)/devices 50 and server computer(s) 60 provide processing, storage, and input/output devices executing application programs and the like. Client computer(s)/devices 50 can also be linked through communications network 70 to other computing devices, including other client devices/processes 50 and server computer(s) 60. Communications network 70 can be part of a remote access network, a global network (e.g., the Internet), a worldwide collection of computers, Local area or Wide area networks, and gateways that currently use respective protocols (TCP/IP, Bluetooth, etc.) to communicate with one another. Other electronic device/computer network architectures are suitable.

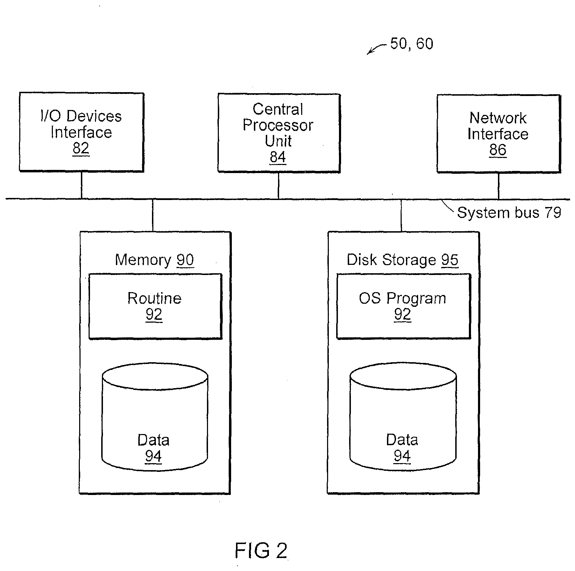

FIG. 2 is a diagram of the internal structure of a computer (e.g., client processor/device 50 or server computers 60) in the computer system of FIG. 1. Each computer 50, 60 contains system bus 79, where a bus is a set of hardware lines used for data transfer among the components of a computer or processing system. Bus 79 is essentially a shared conduit that connects different elements of a computer system (e.g., processor, disk storage, memory, input/output ports, network ports, etc.) that enables the transfer of information between the elements. Attached to system bus 79 is I/O device interface 82 for connecting various input and output devices (e.g., keyboard, mouse, displays, printers, speakers, etc.) to the computer 50, 60. Network interface 86 allows the computer to connect to various other devices attached to a network (e.g., network 70 of FIG. 1). Memory 90 provides volatile storage for computer software instructions 92 and data 94 used to implement an embodiment of the present invention. All of the data 94 required for the calculation of the probability of death of the patient (*i.e.*, Eq. 1 detailed above) can be stored in a clinical data system, for example Proton (Clinical Computing, Inc., Cincinnati, OH). This clinical data from various computers on a network can be compiled in a relational database, for example Oracle database (Oracle Corp., Redwood Shores, CA). A program such as Microsoft Access can be used to extract required clinical data from the Oracle database and perform required calculations. Alternatively, the data extracted in the Microsoft Access can be exported to a spreadsheet program, such as Microsoft Excel, to perform the required calculations. Disk storage 95 provides non-volatile storage for computer software instructions 92 and data 94 used to implement an embodiment of the present invention. Central processor unit 84 is also attached to system bus 79 and provides for the execution of computer instructions.

In one embodiment, the processor routines 92 and data 94 are a computer program product (generally referenced 92), including a computer readable medium (e.g., a removable storage medium such as one or more DVD-ROM's, CD-ROM's, diskettes, tapes, etc.) that provides at least a portion of the software instructions for the invention system. Computer program product 92 can be installed by any suitable software installation procedure, as is well known in the art. In another embodiment, at least a portion of the software instructions may also be downloaded over a cable, communication and/or wireless connection. In other embodiments, the invention programs are a computer program propagated signal product 107 embodied on a propagated signal on a propagation medium (e.g., a radio wave, an infrared wave, a laser wave, a sound wave, or an electrical wave propagated over a global network such as the Internet, or other network(s)). Such carrier medium or signals provide at least a portion of the software instructions for the present invention routines/program 92.

In alternate embodiments, the propagated signal is an analog carrier wave or digital signal carried on the propagated medium. For example, the propagated signal may be a digitized signal propagated over a global network (e.g., the Internet), a telecommunications network, or other network. In one embodiment, the propagated signal is a signal that is transmitted over the propagation medium over a period of time, such as the instructions for a software application sent in packets over a network over a period of milliseconds, seconds, minutes, or longer. In another embodiment, the computer readable medium of computer program product 92 is a propagation medium that the computer system 50 can receive and read, such as by receiving the propagation medium and identifying a propagated signal embodied in the propagation medium, as described above for computer program propagated signal product.

Generally speaking, the term "carrier medium" or "transient carrier" encompasses the foregoing transient signals, propagated signals, propagated medium, storage medium and the like.

EXEMPLIFICATION

Example 1. Model including absolute values of parameters

A retrospective analysis was conducted of incident in-center hemodialysis (HD) patients in Renal Research Institute (New York, NY) clinics (RRI) starting HD between January 1, 2001, and December 31, 2008. The analysis included patients who survived the first 6 months of HD (5,671 patients). The patients' lab and treatment parameters were recorded as averages of the values measured over the first 6 months of HD. Those parameters were used as independent variables in a logistic regression model. The patients' survival status was noted during months 7 to 12. A logistic regression model with death as the outcome (dependent) variable was constructed. The model was then applied to predict mortality risk in these patients during months 13 to 18, based on averages of lab and treatment parameters over months 7 to 12, and during months 19 to 24, based on averages of lab and treatment parameters over months 13 to 18 from the start of dialysis.

The study showed that of the 5,671 patients that survived the first 6 months of dialysis, 354 died in months 7 to 12. Logistic regression parameter estimates are shown in Table 1, where estimates with a P-value <0.05 are statistically significant, and the reference group was race=black, gender=female, and diabetic status=non-diabetic.

Table 1. Logistic regression model parameter estimates for Example 1

| Parameter | Parameter ($\beta$) Estimate | P-value | Odds Ratio (OR)[#] | 95% Confidence Interval (CI) for Odds Ratio | |
|---|---|---|---|---|---|
| Intercept ($\alpha$) | 3.949 | <0.0001 | | | |
| Age* (years) | 0.026 | <0.0001 | 1.03 | 1.02 | 1.04 |
| Race=white | 0.398 | 0.002 | 1.49 | 1.16 | 1.92 |
| Race=other | -0.168 | 0.493 | 0.85 | 0.52 | 1.37 |
| Gender=male | 0.417 | 0.001 | 1.52 | 1.19 | 1.94 |
| Diabetic=yes | 0.005 | 0.965 | 1.01 | 0.79 | 1.28 |
| Pre-dialysis SBP (mmHg) | -0.013 | <0.0001 | 0.99 | 0.98 | 0.99 |
| Pre-dialysis weight (kg) | -0.017 | <0.0001 | 0.98 | 0.98 | 0.99 |
| Serum albumin (g/dL) | -1.165 | <0.0001 | 0.31 | 0.24 | 0.41 |
| Serum bicarbonate (mEq/L) | 0.039 | 0.097 | 1.04 | 0.99 | 1.09 |
| Hemoglobin (g/dL) | -0.154 | 0.005 | 0.86 | 0.77 | 0.95 |
| Ferritin (per 100 ng/mL) | 0.010 | 0.280 | 1.01 | 0.90 | 1.10 |
| Na gradient (dialysate Na - serum Na) (mmol/L) | 0.052 | 0.005 | 1.05 | 1.02 | 1.09 |
| enPCR (mg/kg/day) | -0.338 | 0.318 | 0.71 | 0.37 | 1.38 |
| eKdrt/V | -0.368 | 0.111 | 0.69 | 0.44 | 1.09 |
| * - at initiation of dialysis Reference group =black, female, non-diabetic # - Odds Ratio = $e^{\beta}$ | | | | | |

A negative parameter value (with a corresponding odds ratio less than 1) indicates an inverse relationship between the respective parameter and the probability of death of the patient.

The resulting logistic regression model was validated in the same data set, predicting survival probabilities over the subsequent six months for the patients with HD survival of 6 months (same cohort as used for model generation), 12 months and 18 months, respectively. Receiver-operator characteristic (ROC) curves were computed to assess the predictive power of the model. As shown in Table 2, validating the model in the same data set as was used for model generation resulted in an area under the ROC curve (AUC) of 0.77, and validating the same model in patients with a HD survival of 12 months and 18 months, respectively, resulted in almost identical AUCs.

Table 2. Predictive ability of logistic regression model of Example 1

| | Survival in months 7 to 12 (same data set) | Survival in months 13 to 18 | Survival in months 19 to 24 |
|---|---|---|---|
| Area under the ROC curve (95% CI) | 0.77 (0.74 to 0.79) | 0.77 (0.74 to 0.80) | 0.74 (0.70 to 0.78) |
| Optimal probability threshold for prediction of death within six months | 5.6% | 4.7% | 3.9% |
| Sensitivity at optimal threshold | 0.76 | 0.71 | 0.67 |
| Specificity at optimal threshold | 0.66 | 0.72 | 0.73 |

The optimal alert thresholds, calculated using the Youden index, are also listed in Table 2. These alert values can be used to notify the clinic and the physician that a given patient is at an increased risk of death. The alert threshold can be selected by a clinician based on the Youden index, or based on other methods or considerations. The ability to alert clinic staff and physicians of patients at higher risk of death is crucial for timely diagnostic and therapeutic interventions. This analysis establishes a model that can be used to predict patient survival status in the following six months. Given the AUC values, this model was used to predict survival in incident HD patients up to two years from the start of dialysis.

Example 2. Six month model including absolute values and changes in parameters

A retrospective analysis was conducted of incident in-center HD patients in RRI clinics starting HD between January 1, 2001, and December 31, 2008. The analysis included patients who survived the first 6 months of HD and had all the parameters listed below measured during that time period (3,010 patients). The patients' demographic data, laboratory and treatment parameters were recorded as averages in month 6 from the start of dialysis. Independent variables included: age, race, gender, diabetic status, pre-dialysis SBP, pre-dialysis weight, pre-dialysis pulse pressure, serum albumin level, neutrophil to lymphocyte ratio, serum sodium level, serum bicarbonate level, hemoglobin, enPCR, eKdrt/V, serum creatinine level, and also slopes of the following variables: pre-dialysis systolic blood pressure, pre-dialysis weight, serum albumin level, and neutrophil to lymphocyte ratio. The slopes were computed between months four to six from the start of dialysis treatments. The slopes indicate a trend in the patients' clinical parameters. The patients' survival status was noted in months seven to nine from the start of dialysis treatments. Among the 3,010 patients included in the analysis, 81 patients died during those three months.

A logistic regression model was constructed using survival status as the dependent variable and the independent variables listed above. The model coefficients are listed in Table 3, where estimates with a P-value < 0.05 are statistically significant, and the reference group was race=white, gender=female, and diabetic status=non-diabetic.

Table 3. Six-month logistic regression model parameter estimates

| Parameter | Parameter ($\beta$) Estimate | P-value | Odds Ratio (OR)# | 95% Confidence Interval (CI) for Odds Ratio | |
|---|---|---|---|---|---|
| Intercept ($\alpha$) | 11.733 | 0.018 | | | |
| Race=black | -0.080 | 0.791 | 0.923 | 0.510 | 1.669 |
| Race=other | 0.144 | 0.729 | 1.155 | 0.511 | 2.611 |
| Gender=male | 0.518 | 0.059 | 1.679 | 0.981 | 2.873 |
| Diabetic=yes | -0.120 | 0.655 | 0.887 | 0.525 | 1.499 |
| Age* (years) | 0.043 | 0.000 | 1.044 | 1.020 | 1.070 |
| Pre-dialysis SBP (mmHg) | 0.001 | 0.947 | 1.001 | 0.978 | 1.025 |
| Pre-dialysis pulse pressure (mmHg) | -0.026 | 0.131 | 0.974 | 0.942 | 1.008 |
| Pre-dialysis weight (kg) | -0.018 | 0.033 | 0.982 | 0.966 | 0.999 |
| Serum albumin (g/dL) | -1.497 | <0.0001 | 0.224 | 0.132 | 0.381 |

(continued)

| Parameter | Parameter (β) Estimate | P-value | Odds Ratio (OR)# | 95% Confidence Interval (CI) for Odds Ratio | |
|---|---|---|---|---|---|
| Neutrophil to lymphocyte ratio | 0.125 | 0.003 | 1.133 | 1.043 | 1.230 |
| Serum sodium (mmol/L) | -0.075 | 0.028 | 0.928 | 0.868 | 0.992 |
| Serum bicarbonate (mEq/L) | 0.049 | 0.224 | 1.050 | 0.971 | 1.135 |
| Hemoglobin (g/dL) | 0.005 | 0.956 | 1.005 | 0.854 | 1.182 |
| Serum creatinine (mg/dL) | -0.005 | 0.934 | 0.995 | 0.876 | 1.130 |
| enPCR (mg/kg/day) | 0.399 | 0.510 | 1.491 | 0.455 | 4.881 |
| eKdrt/V | -1.292 | 0.007 | 0.275 | 0.107 | 0.706 |
| Slope of pre-dialysis SBP | 0.036 | 0.050 | 1.037 | 1.000 | 1.075 |
| Slope of pre-dialysis weight | -0.058 | 0.503 | 0.944 | 0.796 | 1.118 |
| Slope of serum albumin | -0.194 | 0.779 | 0.824 | 0.213 | 3.193 |
| Slope of neutrophil to lymphocyte ratio | -0.072 | 0.432 | 0.931 | 0.778 | 1.114 |
| *- at initiation of dialysis Reference group =white, female, non-diabetic # - Odds Ratio = $e^{\beta}$ | | | | | |

The probability of death was computed for the same population using the parameters listed in Table 3. FIG. 1 illustrates the distribution of probability of death, showing a mean of 2.65%. A receiver operator characteristic (ROC) curve was constructed using the predicted and actual deaths, as shown in FIG. 2. The area under the curve (AUC) result shown in FIG. 2 is AUC=0.842 (P<0.001, 95% CI: 0.80 to 0.89). An optimum "alert threshold" was obtained by calculating the maximum Youden index (YI) defined by

$$YI = sensitivity + specificity -1 \qquad (2)$$

The calculated Youden index was 0.60, with a corresponding alert threshold of a probability of death of 2.6%, resulting in a sensitivity of 0.82 and a specificity of 0.78.

Example 3. Twelve month model including absolute values and changes in parameters

A retrospective analysis was conducted of incident in-center HD patients in RRI clinics starting HD between January 1, 2001, and December 31, 2008. The analysis included patients who survived the first 12 months of HD. The patients were divided into two data sets: a "model" data set comprising 80% of the patients, and a "validation" data set comprising 20% of the patients. The patients were randomly assigned between the two groups (2,645 patients in the model group, 664 in the validation group) using random number generation. The patients' lab and treatment parameters recorded at month 12 were used as independent variables in a logistic regression model. Averages of measurements taken during month 12 were used for those parameters that were measured more than once a month. The dependent variable was the patient's survival probability in months 13 to 18.

Logistic regression was performed using the "model" data set, using the following independent variables, measured at month 12: age, race, gender, diabetic status, pre-dialysis SBP, post-dialysis DBP, post-dialysis weight, inter- dialytic weight change, intra-dialytic change in SBP, pre-dialysis pulse pressure, serum albumin level, enPCR, eKdrt/V, TSAT, serum creatinine level, erythropoietin (EPO) resistance index (ERI), the percent change in months 11 to 12 in serum albumin level, the percent change in months 11 to 12 in pre-dialysis weight, the percent change in months 10 to 12 in pre-dialysis weight, and the percent change in months 7 to 12 in ferritin level. The parameters of the logistic regression model are listed in Table 4, where estimates with a P-value<0.05 are statistically significant, and the reference group was race=white or black, gender=female, and diabetic status=non-diabetic.

Table 4. Twelve-month logistic regression model parameter estimates

| Parameter | Parameter (β) Estimate | P-value | Odds Ratio (OR)[#] | 95% Confidence Interval (CI) for Odds Ratio | |
|---|---|---|---|---|---|
| Intercept (α) | 3.1969 | 0.1911 | | | |
| Age* (years) | 0.0129 | 0.3167 | 1.013 | 0.988 | 1.039 |
| Race=other | -0.7158 | 0.2796 | 0.489 | 0.134 | 1.789 |
| Gender=male | 1.0044 | 0.0019 | 2.73 | 1.447 | 5.15 |
| Diabetic=yes | -0.6583 | 0.0313 | 0.518 | 0.284 | 0.943 |
| Pre-dialysis SBP (mmHg) | 0.0608 | 0.0549 | 1.063 | 0.999 | 1.131 |
| Post-dialysis DBP (mmHg) | -0.0747 | 0.0519 | 0.928 | 0.861 | 1.001 |
| Post-dialysis weight (kg) | -0.00994 | 0.2564 | 0.99 | 0.973 | 1.007 |
| Intra-dialytic change in SBP | -0.043 | 0.1415 | 0.958 | 0.905 | 1.014 |
| Pre-dialysis pulse pressure | -0.0593 | 0.0598 | 0.942 | 0.886 | 1.002 |
| Serum albumin (g/dL) | -1.4739 | 0.0001 | 0.229 | 0.107 | 0.489 |
| enPCR (mg/kg/day) | -0.5546 | 0.4746 | 0.574 | 0.126 | 2.627 |
| eKdrt/V | 0.4722 | 0.3993 | 1.604 | 0.535 | 4.808 |
| TSAT | -0.0251 | 0.0767 | 0.975 | 0.948 | 1.003 |
| Serum creatinine (mg/dL) | -0.0512 | 0.4607 | 0.95 | 0.829 | 1.088 |
| ERI | 0.0213 | 0.1323 | 1.022 | 0.994 | 1.05 |
| Pre-dialysis weight slope (3-month) | 0.2544 | 0.0288 | 1.29 | 1.027 | 1.62 |
| Albumin change (2-month) | -0.0443 | 0.0023 | 0.957 | 0.93 | 0.984 |
| Pre-dialysis weight change (2-month) | -0.2332 | 0.002 | 0.792 | 0.683 | 0.918 |
| Ferritin (6-month average) | 0.000268 | 0.3604 | 1 | 1 | 1.001 |
| *- at initiation of dialysis Reference group =white or black, female, non-diabetic # - Odds Ratio = $e^{\beta}$ | | | | | |

A ROC curve constructed using the validation data set yielded an AUC of 0.72 (95% CI: 0.63 to 0.81).

While this invention has been particularly shown and described with references to example embodiments thereof, it will be understood by those skilled in the art that various changes in form and details may be made therein. The scope of the invention is encompassed by the appended claims.

**Claims**

1.   A computer system for identifying a patient undergoing periodic hemodialysis treatments at increased risk of death, the computer system comprising:

a) a user input means for determining patient data from a user;
b) a digital processor coupled to receive determined patient data from the user input means, wherein the digital processor executes a modeling system in working memory, wherein the modeling system:

i) selects one or more clinical or biochemical parameters selected from a statistically significant percent change in the patient's serum albumin level, a statistically significant percent change in the patient's pre-dialysis weight, and a statistically significant percent change in the patient's ferritin level, while the patient is undergoing periodic hemodialysis treatment, wherein a statistically significant percent change is defined by a P< 0.05;

ii) estimates the probability of death of the patient over a future time interval by a logistic regression model including model coefficients, the model coefficients determined by analyzing data from patients that were undergoing periodic hemodialysis treatments, the analysis including a longitudinal analysis in time on the one or more percent change in the clinical or biochemical parameters of the patients; and

iii) identifies the patient as having an increased risk of death if the probability of death of the patient is greater than a predetermined threshold probability; and

c) an output means coupled to the digital processor, the output means provides to the user the probability of death of the patient over the future time interval while the patient is undergoing periodic hemodialysis treatments.

2. The computer system of Claim 1, wherein the future time interval is in a range of between about one month and about six months.

3. The computer system of either Claim 1 or 2, wherein identifying the patient as having an increased risk of death is accomplished within a sufficient lead time to allow for therapeutic intervention to decrease the patient's risk of death.

4. The computer system of any one of Claims 1 to 3, wherein the predetermined threshold probability is about 2.5% in the future time interval.

5. A computer-implemented method of identifying a patient undergoing periodic hemodialysis treatments at increased risk of death, comprising:

selecting one or more clinical or biochemical parameters selected from a statistically significant percent change in the patient's serum albumin level, a statistically significant percent change in the patient's pre-dialysis weight, and a statistically significant percent change in the patient's ferritin level associated while the patient is undergoing periodic hemodialysis treatment, wherein a statistically significant percent change is defined by a P< 0.05;

estimating the probability of death of the patient over a future time interval by a logistic regression model including model coefficients, the model coefficients determined by analyzing data from patients that were undergoing periodic hemodialysis treatments, the analysis including a longitudinal analysis in time on the one or more percent change in the clinical or biochemical parameters of the patients; and

identifying the patient as having an increased risk of death if the probability of death of the patient over the future time interval is greater than a predetermined threshold probability.

6. The method of Claim 5, wherein the future time interval is in a range of between about one month and about six months.

7. The method of either Claim 5 or Claim 6, wherein identifying the patient as having an increased risk of death is accomplished within a sufficient lead time to allow for therapeutic intervention to decrease the patient's risk of death.

8. The method of any one of Claims 5 to 7, wherein the predetermined threshold probability is about 2.5% in the future time interval.

**Patentansprüche**

1. Computersystem zum Identifizieren eines Patienten, der periodischen Behandlungen der Hämodialyse bei erhöhtem Todesrisiko unterzogen wird, wobei das Computersystem Folgendes umfasst:

a) ein Mittel zur Benutzereingabe zum Bestimmen von Patientendaten von einem Benutzer;
b) einen digitalen Prozessor, der angeschlossen ist, um bestimmte Patientendaten von dem Mittel zur Benutzereingabe zu empfangen, wobei der digitale Prozessor ein Modellierungssystem im Arbeitsspeicher ausführt, wobei das Modellsystem:

i) einen oder mehrere klinische oder biochemische Parameter aus einer statistisch signifikanten prozentualen Änderung des Serumalbuminspiegels des Patienten, einer statistisch signifikanten prozentualen Änderung des Vordialysegewichts des Patienten und einer statistisch signifikanten prozentualen Änderung des Ferritinspiegels des Patienten auswählt, während der Patient einer regelmäßig wiederkehrenden Behandlung der Hämodialyse unterzogen wird, wobei eine statistisch signifikante prozentuale Änderung festgelegt ist durch einen $P < 0,05$;

ii) die Wahrscheinlichkeit des Todes des Patienten über ein zukünftiges Zeitintervall mit Hilfe eines logistischen Modellkoeffizienten einschließenden Regressionsmodells schätzt, wobei die Modellkoeffizienten durch Analysieren von Daten von Patienten bestimmt werden, die regelmäßigen Behandlungen der Hämodialyse unterzogen werden, wobei die Analyse eine longitudinale Zeitanalyse an einer oder mehreren prozentualen Änderungen in den klinischen oder biochemischen Parametern der Patienten einschließt; und

iii) den Patienten identifiziert, der ein erhöhtes Todesrisiko aufweist, wenn die Wahrscheinlichkeit des Todes des Patienten größer ist als eine vorbestimmte Schwellenwahrscheinlichkeit; und

c) ein Ausgabemittel, das an dem digitalen Prozessor angeschlossen ist, wobei das Ausgabemittel dem Benutzer die Wahrscheinlichkeit des Todes des Patienten über das zukünftige Zeitintervall bereitstellt, während dem der Patient regelmäßigen Behandlungen der Hämodialyse unterzogen wird.

2. Computersystem nach Anspruch 1, wobei das zukünftige Zeitintervall in einem Bereich zwischen etwa einem Monat und etwa sechs Monaten liegt.

3. Computersystem nach Anspruch 1 oder 2, wobei das Identifizieren des Patienten, der ein erhöhtes Todesrisiko aufweist, innerhalb einer ausreichenden Vorlaufzeit erreicht wird, um einen therapeutischen Eingriff zur Verminderung des Todesrisikos zu ermöglichen.

4. Computersystem nach einem der Ansprüche 1 bis 3, wobei die vorbestimmte Schwellenwahrscheinlichkeit in dem zukünftigen Zeitintervall etwa 2,5% beträgt.

5. Computer implementiertes Verfahren zum Identifizieren eines Patienten, der regelmäßigen Behandlung der Hämodialyse mit erhöhtem Todesrisiko unterzogen wird, umfassend:

Auswählen von einem oder mehreren klinischen oder biochemischen Parametern mit einer Todwahrscheinlichkeit aus einer statistisch signifikanten prozentualen Änderung des Serumalbuminspiegels des Patienten, einer statistisch signifikanten prozentualen Änderung des Vordialysegewichts des Patienten und einer statistisch signifikanten prozentualen Änderung des Ferritinspiegels des Patienten auswählt, während der Patient einer regelmäßig wiederkehrenden Behandlung der Hämodialyse unterzogen wird, wobei eine statistisch signifikante prozentuale Änderung festgelegt ist durch einen $P < 0,05$;

Schätzen der Wahrscheinlichkeit des Todes des Patienten über ein zukünftiges Zeitintervall mit Hilfe eines logistischen, Modellkoeffizienten einschließenden Regressionsmodells, wobei die Modellkoeffizienten durch Analysieren von Daten von Patienten bestimmt werden, die regelmäßigen Behandlungen der Hämodialyse unterzogen werden, wobei die Analyse eine longitudinale Zeitanalyse an einer oder mehreren prozentualen Änderungen in den klinischen oder biochemischen Parametern der Patienten einschließt; und

Identifizieren des Patienten, der ein erhöhtes Todesrisiko aufweist, wenn die Wahrscheinlichkeit des Todes des Patienten größer ist als eine vorbestimmte Schwellenwahrscheinlichkeit.

6. Verfahren nach Anspruch 5, wobei das zukünftige Zeitintervall in einem Bereich zwischen etwa einem Monat und etwa sechs Monaten liegt.

7. Verfahren nach Anspruch 5 oder 6, wobei das Identifizieren des Patienten, der ein erhöhtes Todesrisiko aufweist, innerhalb einer ausreichenden Vorlaufzeit erreicht wird, um einen therapeutischen Eingriff zur Verminderung des Todesrisikos zu ermöglichen.

8. Verfahren nach einem der Ansprüche 5 bis 7, wobei die vorbestimmte Schwellenwahrscheinlichkeit in dem zukünftigen Zeitintervall etwa 2,5% beträgt.

**Revendications**

1. Système informatique destiné à identifier si un patient sous traitements d'hémodialyse périodique présente un risque accru de décès, le système informatique comprenant :

a) un moyen d'entrée d'utilisateur permettant de déterminer des données de patient en provenance d'un utilisateur ;
b) un processeur numérique couplé pour recevoir des données de patient déterminées depuis le moyen d'entrée d'utilisateur, le processeur numérique exécutant un système de modélisation dans une mémoire de travail, où le système de modélisation :

i) sélectionne un ou plusieurs paramètres cliniques ou biochimiques choisis parmi une variation statistiquement significative en pourcentage du niveau d'albumine dans le sérum du patient, une variation statistiquement significative en pourcentage du poids du patient avant dialyse et une variation statistiquement significative en pourcentage du niveau de ferritine chez le patient, pendant que le patient subit un traitement d'hémodialyse périodique, une variation statistiquement significative en pourcentage étant définie par P < 0,05 ;
ii) estime la probabilité de décès du patient sur un intervalle de temps à venir par un modèle de régression logistique incluant des coefficients de modèle, les coefficients de modèle étant déterminés par analyse des données de patients qui subissaient des traitements d'hémodialyse périodique, l'analyse incluant une analyse longitudinale dans le temps de l'au moins une variation en pourcentage des paramètres cliniques ou biochimiques des patients ; et
iii) identifie le patient comme ayant un risque accru de décès si la probabilité de décès du patient est supérieure à un seuil de probabilité prédéterminé ; et

c) un moyen de sortie couplé au processeur numérique, le moyen de sortie fournissant à l'utilisateur la probabilité de décès du patient sur l'intervalle de temps à venir pendant que le patient subit des traitements d'hémodialyse périodique.

2. Système informatique selon la revendication 1, dans lequel l'intervalle de temps à venir est dans la gamme entre environ un mois et environ six mois.

3. Système informatique selon la revendication 1 ou 2, dans lequel identifier le patient comme ayant un risque accru de décès se fait sous un délai de réalisation suffisant pour permettre à une intervention thérapeutique de réduire le risque de décès du patient.

4. Système informatique selon l'une quelconque des revendication 1 à 3, dans lequel le seuil de probabilité prédéterminé est d'environ 2,5 % dans l'intervalle de temps à venir.

5. Procédé mis en oeuvre par informatique d'identification d'un patient subissant des traitements d'hémodialyse périodique présentant un risque accru de décès, comprenant :

le choix d'au moins un paramètre clinique ou biochimique choisi parmi une variation statistiquement significative en pourcentage du niveau d'albumine dans le sérum du patient, une variation statistiquement significative en pourcentage du poids du patient avant dialyse et une variation statistiquement significative en pourcentage du niveau de ferritine chez le patient, pendant que le patient subit un traitement d'hémodialyse périodique, une variation statistiquement significative en pourcentage étant définie par P < 0,05 ;
l'estimation de la probabilité de décès du patient sur un intervalle de temps à venir par un modèle de régression logistique incluant des coefficients de modèle, les coefficients de modèle étant déterminés par analyse des données de patients qui subissaient des traitements d'hémodialyse périodique, l'analyse incluant une analyse longitudinale dans le temps pendant l'au moins une variation en pourcentage des paramètres cliniques ou biochimiques des patients ; et
l'identification du risque accru de décès du patient si la probabilité de décès du patient sur l'intervalle de temps à venir est supérieure à un seuil prédéterminé de probabilité.

6. Procédé selon la revendication 5, dans lequel l'intervalle de temps à venir est dans la gamme entre environ un mois et environ six mois.

7. Procédé selon la revendication 5 ou 6, dans lequel l'identification du risque accru de décès du patient se fait en un délai de réalisation suffisant pour permettre à une intervention thérapeutique de réduire le risque de décès du patient.

8. Procédé selon l'une quelconque des revendication 5 à 7, dans lequel le seuil prédéterminé de probabilité est d'environ 2,5 % dans l'intervalle de temps à venir.

FIG 1

~50, 60

| | | |
|---|---|---|
| I/O Devices Interface 82 | Central Processor Unit 84 | Network Interface 86 |

System bus 79

| Memory 90 | Disk Storage 95 |
|---|---|
| Routine 92 | OS Program 92 |
| Data 94 | Data 94 |

FIG 2

FIG. 3

FIG. 4

## REFERENCES CITED IN THE DESCRIPTION

**Patent documents cited in the description**

- US 58794109 A

**Non-patent literature cited in the description**

- United States Renal Data System, USRDS 2009 Annual Data Report. National Institutes of Health
- **D. G. KLEINBAUM ; M. KLEIN.** Survival Analysis. Springer, 2005
- **J. T. DAUGIRDAS ; P. G. BLAKE ; T. S. ING.** *Handbook of Dialysis,* 2007